(19) **Europäisches Patentamt**
**European Patent Office**
**Office européen des brevets**

(11) **EP 2 904 965 B1**

(12) **EUROPEAN PATENT SPECIFICATION**

(45) Date of publication and mention
of the grant of the patent:
**19.07.2017 Bulletin 2017/29**

(51) Int Cl.:
*A61B 5/0408* (2006.01)    *G01R 33/567* (2006.01)
*A61B 5/00* (2006.01)    *A61B 5/0428* (2006.01)
*A61B 5/055* (2006.01)

(21) Application number: **15154089.5**

(22) Date of filing: **06.02.2015**

(54) **Analog cancellation of MRI sequencing noise appearing in an ECG signal**

Analoge Unterdrückung von MRT-Sequenzierungsrauschen in einem EKG-Signal

Suppression analogique du bruit de séquençage d'IRM apparaissant dans un signal ECG

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **07.02.2014 US 201414174987**

(43) Date of publication of application:
**12.08.2015 Bulletin 2015/33**

(73) Proprietor: **Biosense Webster (Israel) Ltd.
2066717 Yokneam (IL)**

(72) Inventors:
• **Govari, Assaf**
**3440001 Haifa (IL)**
• **Ephrath, Yaron**
**3707969 Karkur (IL)**

(74) Representative: **Carpmaels & Ransford LLP
One Southampton Row
London WC1B 5HA (GB)**

(56) References cited:
**EP-A2- 0 470 764    US-A- 4 991 587
US-A- 5 038 785    US-A1- 2013 221 964**

**Description**

**FIELD OF THE INVENTION**

The present invention relates generally to electrocardiograph (ECG) signals, and specifically to detecting the ECG signals during a magnetic resonance imaging (MRI) procedure.

**BACKGROUND OF THE INVENTION**

Magnetic resonance imaging (MRI) is an extremely powerful technique for visualizing tissue, particularly soft tissue, of a patient. The technique relies on exciting nuclei, typically hydrogen nuclei, from their equilibrium state, and measuring the resonant radiofrequency signals emitted by the nuclei as they relax back to equilibrium.

In an MRI procedure the cardiac condition of a patient placed in the rapidly changing magnetic fields generated by the procedure may need to be assessed. Monitoring of an electrocardiograph (ECG) signal is a good indicator of the cardiac condition of the patient, so that ECG monitoring may enhance the efficacy of the procedure.

U. S. Patent 6,873,869, to Fischer, describes a method of obtaining an ECG signal of a patient located in a turbulent electromagnetic environment. The signal, including added noise, is recovered near the cardiac region as a differential signal resulting from signals delivered by two electrodes forming part of a first measurement loop.

U. S. Patent 5,217,010, to Tsitlik, et al., describes a device for monitoring a patient or pacing a patient which is stated to be able to safely operate in an MRI system. The device claims to use unique RF filtering and shielding to attenuate voltages on the leads resulting from the high frequency RF signals produced in the MRI.

U. S. Patent Application 2004/0225210, to Brosovich, et al., describes a lead set assembly which is stated to be capable of coupling electrodes to a separate device. The assembly includes two leads. A condition sense lead features two conductive paths, each being connectible to one electrode for conveying a bioelectric signal therefrom and which is capable of having a first noise signal induced therein by electromagnetic emanations. A noise pickup lead also features two conductive paths, each being capable of having a second noise signal induced therein by the electromagnetic emanations.

U. S. Patent 7,039,455, to Brosovich, et al., describes apparatus for improving the quality of ECG signals obtained from a patient undergoing MRI. The apparatus includes the arrangement of a differential amplifier, a prefilter, a signal limiter circuit and an intermediate amplifier with an integral low pass filter.

U. S. Patent 7,286,871, to Cohen, describes a method of reducing contamination of electrical signals recorded in the presence of repeated interference contamination The electrical signal is digitized, the digitizing beginning with a timing signal. A plurality of digitized electrical signals is analyzed, and the electrical signals are synchronized with respect to the timing signal, to obtain an estimated contaminating signal that is subtracted from the digitized electrical signal.

U. S. Patent 4,991,580, to Moore, describes a method for improving the quality of ECG signals obtained from a patient undergoing MRI. The method includes conducting the ECG signals having MRI induced noise signals to the input of a slew rate limiter (SRL) circuit having a preselected maximum slew rate. The output of the SRL circuit is connected to a low pass filter circuit.

U. S. Patent 6,070,097, to Kreger, et al., describes a method for generating a gating signal for cardiac MRI. An MRI system includes a detector system which receives an ECG signal from a patient being scanned and produces the gating signal. The gating signal is produced when a detected peak in the ECG signal meets a set of R-wave criteria.

European Patent 1,872,715, to Uutela Kimmo, describes reference data indicative of statistical properties of biosignal artifacts that is generated by a turbulent electromagnetic environment and that is collected. Multiple channels of the biosignal of the patient are measured in the turbulent electromagnetic environment. Artifacts in the multiple channels are detected using the reference data and parameters are derived for a linear combination of the multiple channels. A refined biosignal is obtained by applying a linear combination to desired signal samples of the multiple channels, the linear combination being defined by the parameters derived.

PCT application WO/2012/170119, to Schweitzer et al., describes a system for tracking catheter electrode locations with the body of a patient during an MRI scan sequence The system includes mitigation logic configured to identify one or more impedance measurements that were taken during potentially noise-inducing conditions, and were thus subject to corruption by noise. The mitigation logic is configured to replace the potentially corrupt impedance measurements with previously-obtained impedance measurements taken from an immediately preceding acquisition cycle.

An article titled "Cardiac MRI: Part 2, Pericardial Diseases" by Prabhakar Rajiah, in Volume 197, Issue 4 of the American Journal of Roentgenology, published in October 2011, describes the use of MRI in cardiac procedures. U.S. Patent 4,991,587, to Blakeley et al., describes adaptive filtering of physiological signals in physiologically gated magnetic resonance imaging.

**SUMMARY OF THE INVENTION**

An embodiment of the present invention provides a method, including:

while imaging a patient using an initial magnetic resonance imaging (MRI) sequence, generating an initial MRI reference signal in response to the initial MRI sequence using a reference sensor placed in proximity to the patient;
identifying, by locking on to the initial MRI reference signal, noise that will be generated in electrocardiograph (ECG) signals received from the patient;
computing a programmable correction to be applied to the ECG signals to reduce the noise; and
applying the programmable correction to reduce the noise in the ECG signals, received from the patient, while imaging the patient using a subsequent MRI sequence and while locking on to a subsequent MRI reference signal generated in response to the subsequent MRI sequence using the reference sensor.

Typically the method includes placing the reference sensor so as not to pick up the ECG signals.

In a disclosed embodiment the reference sensor consists of an antenna.

In a further disclosed embodiment the programmable correction includes a set of frequency dependent gains.

In a yet further disclosed embodiment identifying the noise consists of analyzing the ECG signals generated from stationary electrodes coupled to the patient.

In an alternative embodiment identifying the noise includes comparing the ECG signals received from the patient while not imaging the patient with an MRI sequence to the ECG signals received from the patient while imaging the patient using the initial MRI sequence.

In a further alternative embodiment identifying the noise includes summing the ECG signals received from the patient while imaging the patient using the initial MRI sequence. Typically, summing the ECG signals consists of summing the ECG signals generated from stationary electrodes coupled to the patient.

There is further provided, according to an embodiment of the present invention, apparatus, including:

a reference sensor, placed in proximity to a patient, and configured, while the patient is imaged using an initial magnetic resonance imaging (MRI) sequence, to generate an initial MRI reference signal in response to the initial MRI sequence; and
a processor, which is configured:

to identify, by locking on to the initial MRI reference signal, noise that will be generated in electrocardiograph (ECG) signals received from the patient,
to compute a programmable correction to be applied to the ECG signals to reduce the noise, and
to apply the programmable correction to reduce the noise in the ECG signals, received from the patient, while imaging the patient using a subsequent MRI sequence and while locking on to a subsequent MRI reference signal generated in response to the subsequent MRI sequence using the reference sensor.

The present invention will be more fully understood from the following detailed description of the embodiments thereof, taken together with the drawings in which:

**BRIEF DESCRIPTION OF THE DRAWINGS**

Fig. 1 is a schematic, pictorial illustration of a system for analog cancellation of magnetic resonance imaging (MRI) noise appearing in electrocardiograph (ECG) signals, according to an embodiment of the present invention;
Fig. 2 is a set of voltage vs. time and magnetic field vs. time graphs schematically illustrating a sequence of MRI signals generated during an MRI procedure, according to an embodiment of the present invention;
Fig. 3 is an alternative set of voltage vs. time and magnetic field vs. time graphs schematically illustrating a sequence of MRI signals generated during an MRI procedure, according to an embodiment of the present invention;
Fig. 4 is a flowchart of steps performed in implementing the system of Fig. 1, according to an embodiment of the present invention;
Fig. 5 is a flowchart of steps performed in implementing the system of Fig. 1, according to an alternative embodiment of the present invention; and
Fig. 6 is a flowchart of steps performed in implementing the system of Fig. 1, according to a further alternative embodiment of the present invention.

**DETAILED DESCRIPTION OF EMBODIMENTS**

OVERVIEW

An embodiment of the present invention reduces magnetic resonance imaging (MRI) noise that is picked up in electrocardiograph (ECG) signals received from a patient, while the patient is imaged by an MRI sequence. In order to reduce the noise a sensor, typically an antenna, that is able to detect MRI radiation from an MRI scanner, is positioned in proximity to the patient. The sensor is typically positioned so that it does not pick up ECG signals from the patient, but so that it produces a signal, used as an MRI reference signal, that supports a processor in reducing the MRI noise.

In some embodiments the noise is first detected and characterized using a training phase. The training phase is followed by an operational phase, wherein a correction factor generated in the training phase is applied

In the training phase, ECG signals from the patient are first recorded while the MRI sequence is not operative. These signals are used as ECG reference signals. The MRI sequence is then activated, so generating the MRI reference signal described above, as well as introducing MRI noise into the ECG signals. The processor uses the MRI reference signal to lock onto the ECG signals, and compares the reference ECG signals with those generated while being locked on. Using the comparison, the processor generates a correction factor, typically a set of frequency dependent gains which may be applied to the ECG signals so as to reduce the MRI noise.

Respective correction factors may be generated for different types of MRI sequence, as well as for variations of defining parameters of each type of sequence.

In the operational phase, a correction factor is applied to subsequent ECG signals generated during subsequent MRI sequences, while locking on to the MRI reference signal generated during activation of the sequence. Typically the type of sequence, as well as the parameters defining the sequence, may be identified from the MRI reference signal.

In alternative embodiments of the present invention, there is no training phase. Rather, the introduced MRI noise is detected, and reduced, during the operational phase, using methods similar to those described above. The detection and reduction may be performed in an "on-the-fly" manner, using iteration to improve the detection and reduction of the injected MRI noise.

DETAILED DESCRIPTION

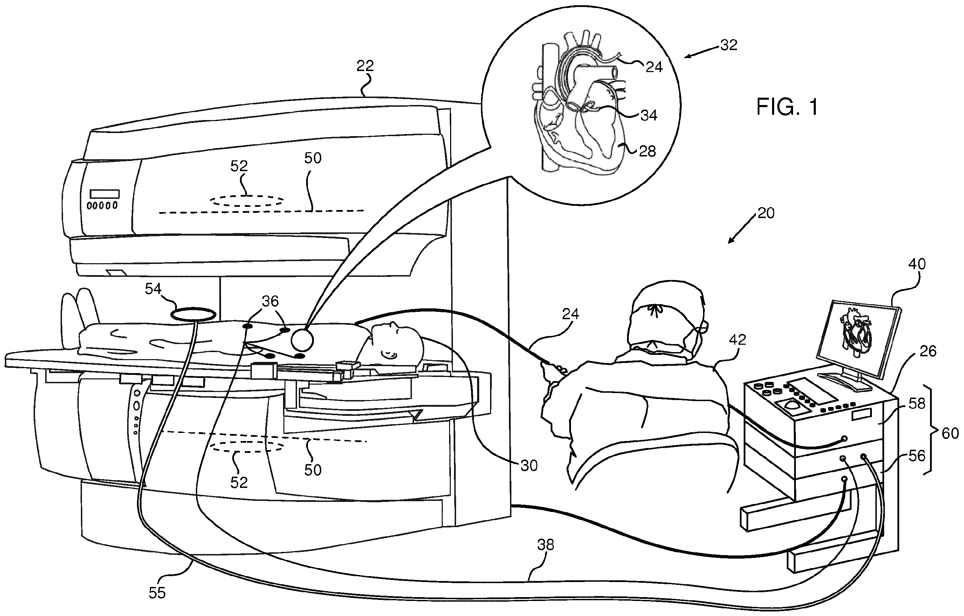

Reference is now made to Fig. 1, which is a schematic, pictorial illustration of a system 20 for analog cancellation of magnetic resonance imaging (MRI) sequencing noise appearing in electrocardiograph (ECG) signals, according to an embodiment of the present invention. System 20 comprises an MRI scanner 22, a probe 24, such as a catheter, and a control console 26. Probe 24 may be used for acquiring ECG signals in a chamber of a heart 28 of a patient 30, using one or more electrodes 32 in a distal end 34 of the probe. Signals acquired by electrodes 32 are herein termed internal ECG signals. In some embodiments, probe 24 may be used for additional purposes, such as for performing cardiac ablation. Further alternatively, probe 24 may be used, *mutatis mutandis,* for other therapeutic and/or diagnostic functions in the heart or in other body organs. The internal ECG signals acquired by electrodes 32 are transferred, typically by conductors and/or optical fibers in probe 24, to control console 26, wherein the signals may be analyzed.

In addition to acquiring internal ECG signals using probe 24, system 20 typically also acquires ECG signals from the skin of patient 30, typically by placing a number of conductive patches 36, which act as electrodes, on the skin of the patient. Signals acquired by patches 36 are herein termed external ECG signals. External ECG signals are conveyed via a cable 38 to control console 26, which analyzes the signals. Results derived from the analysis may be presented on a display 40 to an operator 42 of system 20.

Operator 42, typically a cardiologist, inserts probe 24 through the vascular system of patient 30 so that distal end 34 of the probe enters a body cavity, herein assumed to be the cardiac chamber from where the internal ECG signals are acquired. Typically the distal end of the probe is tracked by a method known in the art. Magnetic position tracking techniques are described, for example, in U.S. Patents 5,391,199, 5,443,489, 6,788,967, 6,690,963, 5,558,091, 6,172,499 6,177,792, whose disclosures are incorporated herein by reference. Impedance-based position tracking techniques are described, for example, in U.S. Patents 5,983,126, 6,456,864 and 5,944,022, whose disclosures are also incorporated herein by reference.

MRI scanner 22 comprises magnetic field coils 50, including field gradient coils, which together generate a spatially variant magnetic field B(x,y,z). The spatially variant magnetic field provides spatial localization for radio frequency (RF) signals generated in the scanner. In addition, the scanner comprises transmit/receive coils 52. In a transmit mode coils 52 radiate RF pulsed energy to patient 30, the RF pulses of energy interacting with the nuclear spins of the patient's tissue and thereby realigning the magnetic moments of the nuclei away from their equilibrium positions. In a receive mode, coils 52 detect RF signals received from the patient's tissue as the tissue nuclei relax to their equilibrium state. The frequency of the signals generated by the relaxation of nuclei in a given region, the Larmor frequency, is directly proportional to the magnetic field at the region, with a constant of proportionality given by the gyromagnetic ratio y of the nuclei. Thus, for hydrogen nuclei, equation (1) applies:

$$f(x, y, z) = \frac{\gamma}{2\pi} \cdot B(x, y, z) \qquad (1)$$

where f(x,y,z) is the frequency radiated by the relaxing hydrogen nuclei from a point (x,y,z), B(x,y,z) is the magnetic field at the point, and $\dfrac{\gamma}{2\pi}$ is equal to approximately 42.6 MHz·T$^{-1}$.

System 20 also comprises an MRI reference sensor 54, typically an antenna, and which may also be referred to herein as antenna 54. Antenna 54 generates a signal in response to operation of scanner 22, by picking up MRI radiation from coils 50 and 52. Antenna 54 is connected by a cable 55, typically a coaxial cable, to console 26. As is described later on, system 20 uses the antenna signal, typically after it has been transferred through an isolating differential amplifier (not shown) as an MRI reference signal. While sensor 54 is typically an antenna, the scope of the present invention includes any type of sensor, such as a semiconductor sensor, that is able to generate a signal by picking up MRI radiation.

In some embodiments, scanner 22 is operated by a scanner processor 56, and the ECG and MRI reference signals described above are analyzed by an ECG processor 58. Processor 58 typically also tracks the probe acquiring the internal ECG signals. For simplicity, in the description herein a single processor 60 in console 26 is assumed to operate system 20, and those having ordinary skill in the art will be able to adapt the description in the event that more than one processor operates the system.

Thus, in addition to analyzing the ECG and MRI reference signals referred to above, and tracking the probe acquiring the internal ECG signals, processor 60 operates scanner 22 by using circuitry to control coils 50, including forming required magnetic field gradients, as well as other circuitry to operate transmit/receive coils 52.

Processor 60 typically comprises a general-purpose computer, which is programmed in software to carry out the functions that are described herein. The software may be downloaded to processor 60 in electronic form, over a network, for example, or it may be provided on non-transitory tangible media, such as optical, magnetic or electronic memory media. Alternatively, some or all of the functions of processor 60 may be carried out by dedicated or programmable digital hardware components, or by using a combination of hardware and software elements.

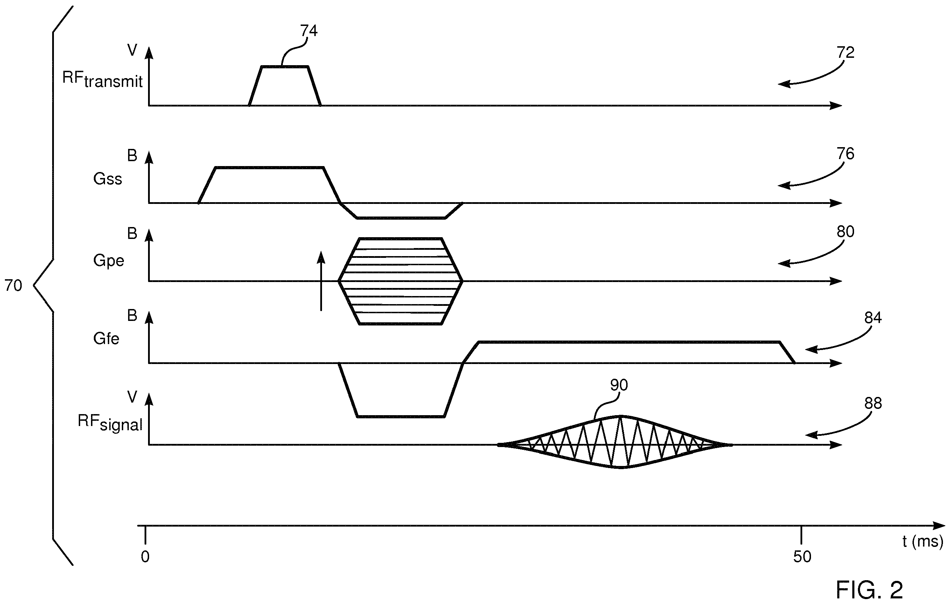

Fig. 2 is a first set of voltage (V) vs. time (t) and magnetic field (B) vs. time (t) graphs schematically illustrating a sequence 70 of MRI signals generated during an MRI procedure, according to an embodiment of the present invention. A first voltage vs. time graph 72 illustrates a transmit RF pulse 74, generated by coils 52, which is transmitted at the start of sequence 70. The transmit RF pulse length is typically of the order of 2 ms, although it may be larger or smaller than this. Encompassing the transmit RF pulse, illustrated in a first magnetic field vs. time graph 76, is a slice selection gradient (Gss) magnetic field pulse generated by coils 50. The slice selection gradient field identifies a volume of interest in patient 30 that is to be imaged by scanner 22.

A second magnetic field vs. time graph 80 illustrates a phase encoding gradient (Gpe) field pulse, which selects a vertical position of points within the volume of interest. A third magnetic field vs. time graph 84 illustrates a frequency encoding gradient (Gfe) field pulse, which selects a horizontal position of points within the volume of interest.

A second voltage vs. time graph 88 illustrates a receive RF pulse 90, corresponding to the data acquisition signal received by coils 52 in response to the transmit RF pulse.

A typical time between the center of the RF transmit pulse and the center of the RF receive pulse may be approximately 30 ms. A typical time for the overall sequence may be approximately 40 ms. However, the actual times may be larger or smaller than these values.

During the course of an MRI procedure, the sequence of RF pulses and magnetic field settings, illustrated by sequence 70, may be repeated, typically at a repetition rate of the order of 1 s. At each repetition of sequence 70, one or more of the variables defining the magnetic field pulses may be varied, typically so that different regions of patient 36 may be scanned. In some cases, as sequence 70 is repeated, variables of RF transmit pulse 74, such as its amplitude, frequency, or phase, may be changed.

Sequence 70 illustrates one type of MRI sequence, termed a gradient echo sequence, that is typically used in an MRI procedure.

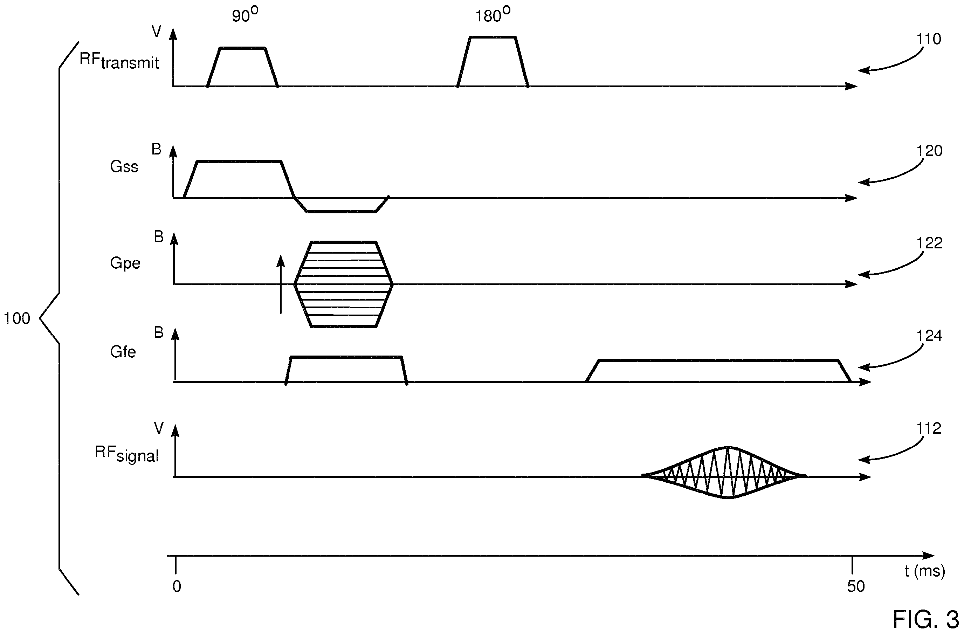

Fig. 3 is a second set of voltage vs. time and magnetic field vs. time graphs schematically illustrating a sequence 100 of MRI signals generated during an MRI procedure, according to an embodiment of the present invention. As for sequence 70, sequence 100 comprises transmit and receive voltage vs. time graphs 110 and 112, and three magnetic field vs. time graphs 120, 122, and 124. As is illustrated in the graphs, sequence 100 uses two RF transmit pulses and two magnetic field Gfe pulses (in contrast to the one RF pulse and one Gfe pulse of sequence 70). Sequence 100 illustrates a spin echo sequence, that may typically also be used in an MRI procedure.

Figs. 2 and 3 illustrate two types of MRI sequence that may be used in embodiments of the present invention. Variations on these sequences, as well as other possible sequences, will be apparent to those having ordinary skill in the art, and all such sequences are assumed to be within the scope of the present invention. In the following description, an identifier "m" is used to label each type of MRI sequence, and by way of example the MRI sequence illustrated by Fig. 2 is assumed to have identifier m = 1, and the MRI sequence illustrated by Fig. 3 is assumed to have identifier m = 2.

Figs. 2 and 3 also illustrate that each MRI sequence is comprised of a number of phases, corresponding to the different pulses that are generated for the sequence. A phase of an MRI sequence may be defined in terms of a pulse, a number of pulses, and/or parts of a pulse. For example, a selected phase of the MRI sequence of Fig. 2 may be defined as the duration of the Gpe pulse; for the MRI sequence of Fig. 3, another selected phase may be defined as the duration of a first half of the second Gfe pulse.

In general, any MRI sequence may be defined in terms of the variables describing each of the pulses graphed in Figs. 2 and 3. Thus, referring to voltage vs. time graphs 72 and 110, the variables comprise the frequency, phase, and amplitude of the voltage of the RF pulse initiating a sequence, changes in values of these variables over time, variables of any subsequent RF pulses, and timing of the subsequent RF pulses with respect to the initial RF pulse. Referring to magnetic field vs. time graphs 76, 80, and 84, and graphs 120, 122, and 124, the variables include those describing the shape of the magnetic field pulses, i.e., the amplitude of the field as it varies in time, and timing of the magnetic field pulses with respect to the RF pulse or pulses in graphs 72 and 112, and with respect to each other.

More specifically, each MRI sequence m may be characterized broadly in terms of a set of parameters, each parameter corresponding to one of the pulses of the sequence. In the following description, the set of parameters for an MRI sequence m is written as $\{S\}_m$.

For example, MRI sequence m = 1, illustrated in Fig. 2, may have a set of parameters $\{S\}_1 = \{P_{RF}, P_{Gss}, P_{Gpe}, P_{Gfe}, P_{signal}\}$, each of the elements of the set representing a voltage or a magnetic field pulse. In contrast, MRI sequence m = 2, illustrated in Fig. 3, has a set of parameters $\{S\}_2 = \{P_{RF1}, P_{RF2}, P_{Gss}, P_{Gpe}, P_{Gfe1}, P_{Gfe2}, P_{signal}\}$.

For any given MRI sequence m, there is a further, more detailed, characterization of each of the parameters in the set. Thus, referring to voltage vs. time graph 72 of m = 1, parameter $P_{RF}$ is characterized in terms of the variables frequency f, phase $\phi$, and amplitude A of the voltage of the RF pulse initiating a sequence, and changes in values of these variables over time t. $P_{RF}$ may thus be represented as a set of ordered 4-tuples, which may be written as an equation (2):

$$P_{RF} \equiv \{(f, \emptyset, A, t)\}_1 \qquad (2)$$

where the set subscript is the identifier m of the sequence.

Similarly, referring to magnetic field Gss vs. time graph 76 of m = 1, parameter $P_{Gss}$ is characterized in terms of the variable magnetic field B at time t, so that $P_{Gss}$ may be represented as a set of ordered pairs of variables, which may be written as an equation (3):

$$P_{Gss} \equiv \{(B, t)\}_1 \qquad (3)$$

Referring to voltage vs. time graph 110 of m = 2, parameters $P_{RF1}$ and $P_{RF2}$ may be represented by the equations:

$$P_{RF1} \equiv \{(f_1, \emptyset_1, A_1, t)\}_2 \qquad (4)$$

and

$$P_{RF2} \equiv \{(f_2, \emptyset_2, A_2, t)\}_2 \qquad (5)$$

where $f_1$, $\phi_1$, $A_1$ are the frequency, phase and amplitude variables at time t of the first pulse of m = 2, and $f_2$, $\phi_2$, $A_2$ are the frequency, phase and amplitude variables at time t of the second pulse of m = 2.

As another example, referring to field vs. time graph 124, parameters $P_{Gfe1}$, $P_{Gfe2}$ may be represented by the equations:

$$P_{Gfe1} \equiv \{(B_1, t)\}_2 \tag{6}$$

and

$$P_{Gfe2} \equiv \{(B_2, t)\}_2 \tag{7}$$

where $B_1$ and $B_2$ are the field variables of the first Gfe pulse and the second Gfe pulse at time t.

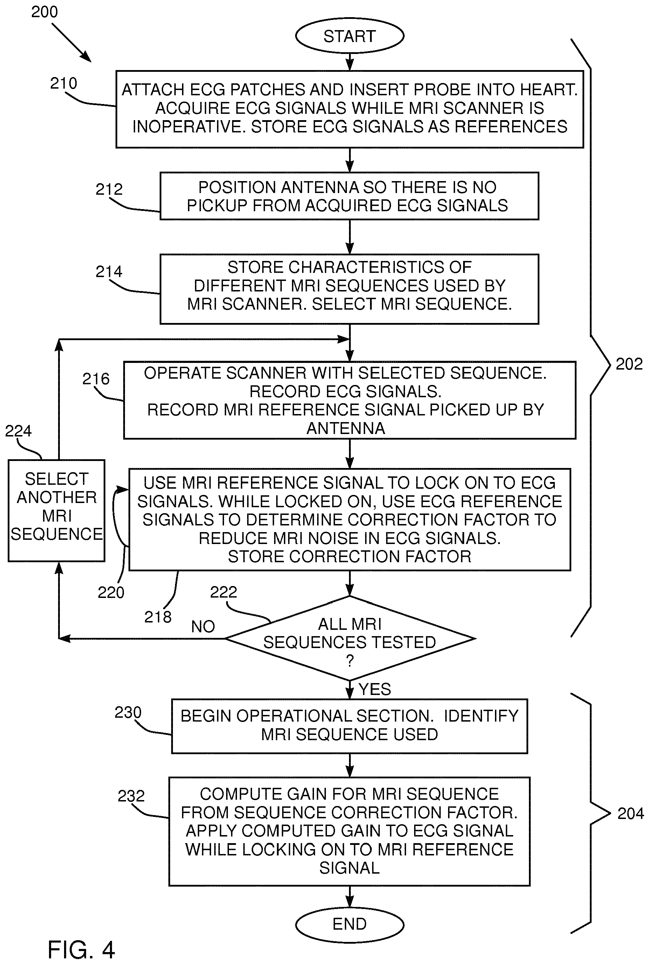

Prior to operating scanner 22, operator 42 uses processor 60 to store $\{S\}_m$, in terms of its parameters and the variables associated with each parameter (in the broad and more detailed manner described above) for each MRI sequence m. During a procedure where scanner 22 is operated, the processor recalls the parameters and variables of $\{S\}_m$ as is explained below with reference to the flowchart of Fig. 4. A specific set of numerical values defining the values of the parameters and variables of $\{S\}_m$ is referred to hereinbelow as $\{S\}_{mp}$, where p is an index representing the numerical values.

Fig. 4 is a flowchart 200 of steps performed in implementing system 20, according to an embodiment of the present invention. The flowchart is divided into two sections: a training phase or section 202 and an operational phase or section 204. The description of the flowchart assumes that appropriate steps of the flowchart are implemented using single processor 60. If system 20 comprises scanner processor 56 and ECG processor 58, then the flowchart steps may be assumed to be implemented by ECG processor 58, using communications as to the timing and identity of MRI sequence m, and as to the numerical values p of the sequence, from scanner processor 56.

In an initial step 210 of the training phase, operator 42 attaches patches 36 to patient 30, and inserts probe 24 into heart 28 of the patient. Patches 36 acquire external ECG signals, and the electrode or electrodes of probe 24 acquire internal ECG signals, while scanner 22 is inoperative. Processor 60 receives the acquired ECG signals, and stores a selected group of the signals as a reference set of signals.

Typically, the reference set of signals comprises signals from electrodes which are relatively stationary with respect to patient 30. For example, the reference set of signals may be from patches 36. In some embodiments probe 24 may be stationary, e.g., if it is being used as an internal reference probe in heart 28, in which case other movable probes (not shown in Fig. 1) may be inserted into heart 28. If probe 24 is stationary, then signals from its electrode or electrodes may be used as ECG reference signals. In some embodiments the reference set may comprise a sub-set of patch signals, such as a selected one of the signals from the patches, and/or a sub-set of signals from electrodes of probe 24 if the probe is stationary.

In an antenna setup step 212, antenna 54 is positioned in proximity to patient 30, typically above a covering of the patient. The location and orientation of the antenna are chosen so that there is no pickup by the antenna of ECG signals generated by the patient. For example, the antenna may be positioned above the patient's abdominal area.

In a characterization step 214, the processor stores the parameters and the variables of the different MRI sequences to be used in operating system 20. In other words, the processor stores the sets $\{S\}_m$ for each value of m to be used in the procedure. Once the parameters and the variables have been stored the processor selects an MRI sequence m with which to operate the scanner, and a set $\{S\}_{mp}$ of numerical values for the sequence.

In a recording step 216, scanner 22 is operated using the selected MRI sequence, and the processor acquires and records ECG signals, from patches 36 and from the electrodes of probe 24, generated while the scanner operates. The recorded ECG signals are selected to correspond with, i.e., to be on the same leads as, the reference signals stored in step 210. The recorded ECG signals include noise, injected, *inter alia,* into the leads conveying the signals, because of the scanner operation. The scanner operation is typically for a multiplicity of scans of the sequence, although there is no requirement for this, and the operation may be for single scan of the sequence.

In addition, while acquiring and recording the ECG signals, and while scanner 22 is being operated, in step 216 the processor also acquires and records the signal generated in sensor 54, the MRI reference signal.

In a correction determining step 218, the recorded ECG signals of step 216 are compared with the reference set of ECG signals stored in step 210, while locking onto the MRI reference signal. From the comparison, the processor determines a correction factor to be applied to the recorded ECG signals so that the resultant ECG signals approximate to the reference signals. The correction factor is typically a set of gains $\{G\}$ which are frequency dependent, and which are applied by the processor to the recorded ECG signals, although in some embodiments the correction factor may be a single value gain. There is a set of gain values for each MRI sequence m, and for the set of numerical values $\{S\}_{mp}$ associated with the m sequence.

Using tested p values processor generates, typically by interpolation and/or extrapolation, an expression, $\{G\}_{mp}$, for the correction factor to be applied for any sequence m and for any numerical value p within the sequence, as given by equation (8).

$${G}_{mp} = {(G_i, f_i}_{mp} \qquad\qquad (8)$$

where $G_i$ is the gain applied at frequency $f_i$, and where i is an identifier of the frequency.

It will be understood that ${G}_{mp}$ represents the set of gains and associated frequencies required to reduce the noise generated by MRI sequence m for the sequence numerical values having an index p.

Typically, as shown by an arrow 220, for a given value of m step 218 is repeated a number of times, for different values of p, so that the values of the specific gains $G_i$ and frequencies $f_i$ in equation (8) may be determined more exactly. The repetition also allows for a range of p values.

In a decision step 222, the processor checks if all MRI sequences have been tested, i.e., if gains for all values of m in equation (8) have been generated. If a sequence remains, the flowchart proceeds to step 224 to select another MRI sequence, and then returns to step 216. If all sequences have been analyzed, training section 202 concludes and operational section 204 begins.

In a first step 230 of the operational section, the processor identifies the MRI sequence m being used. The processor also determines the specific numerical values of ${S}_{mp}$ being used for the sequence. The identification of m and ${S}_{mp}$ may be made by communication from scanner 22. Alternatively or additionally, the processor may analyze characteristics of the MRI reference signal picked up by sensor 54 in order to identify m and the associated numerical values.

In a final step 232 of the operational section, the processor computes the correction factor, i.e., the gains to be applied, from equation (8). The processor then applies the calculated gains to all the ECG signals received from patches 36 and from the electrodes of probe 24, while the MRI sequence is operative, so as to reduce the MRI noise in the signals. The processor determines operation of the MRI sequence by locking on to the MRI reference signal generated in sensor 54.

The description above of flowchart 200 assumes that a complete MRI sequence m is being considered, and that gains given by equation (8) are applied to the ECG signals acquired while the complete sequence is operative. Those having ordinary skill in the art will be able to adapt the description, *mutatis mutandis,* for the case that noise from one or more phases of MRI sequence m is created in the ECG signals, and that the noise for each phase is to be reduced separately. In this case equation (8) may be used to calculate gains to be applied during each separate phase, and the separate phases may be identified, in both the training and operational sections of the flowchart, from the MRI reference signal generated in sensor 54, and/or by communications from scanner 22.

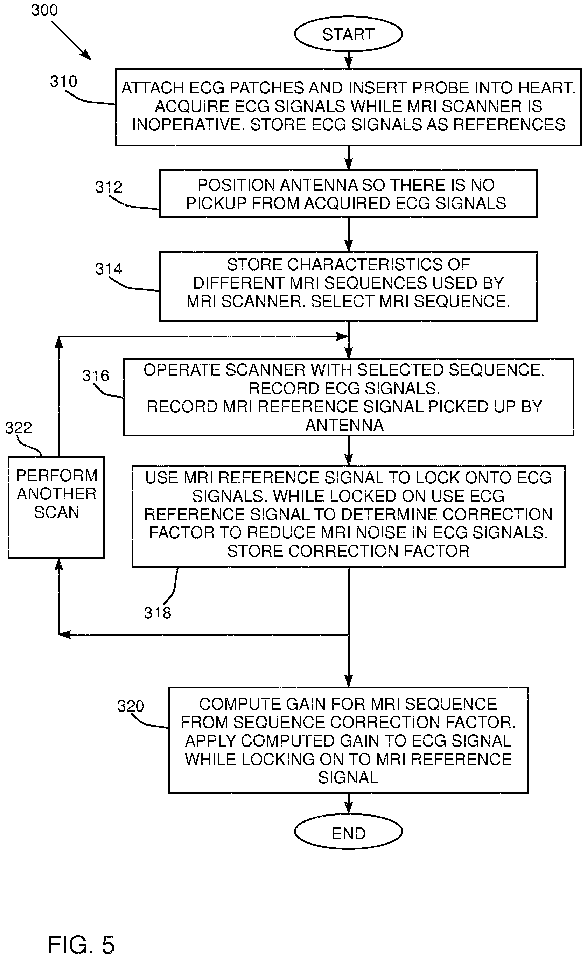

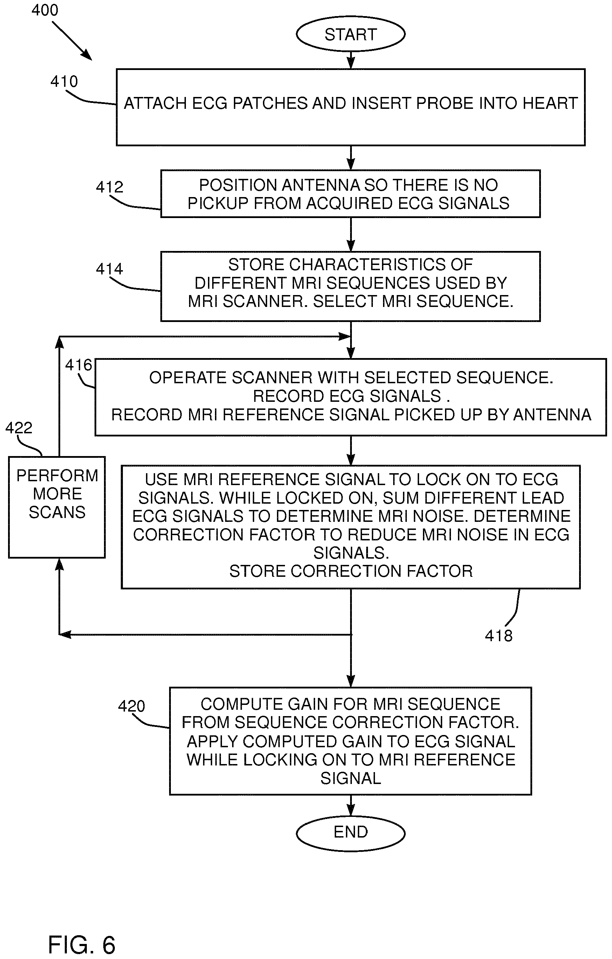

The description of flowchart 200 has also assumed that system 20 is implemented using training section 202 followed by operational section 204. However, in alternative embodiments of the present invention there is no requirement for a separate training section, and in these embodiments the training of system 20 is performed "on-the-fly." The steps of flowcharts 300 and 400, described below with reference to Figs. 5 and 6, exemplify how system 20 may be implemented during performance of an MRI procedure on a patient, without a separate training section.

Fig. 5 is a flowchart 300 of steps performed in implementing system 20, according to an alternative embodiment of the present invention. First, second, and third steps 310, 312, and 314 are substantially as described for steps 210, 212, and 214 of flowchart 200, respectively storing reference ECG signals typically derived from stationary electrodes, positioning antenna 54, and storing MRI sequence characteristics. A recording step 316 is generally the same as step 216. However, typically the ECG recording is for a small number of MRI scans, or for a single scan.

A correction determining step 318 is also generally the same as step 218. Thus, the processor determines a correction factor, typically in terms of gains given by equation (8), that are to be applied to the recorded ECG signals so that the resultant ECG signals approximate to the ECG reference signals.

The flowchart then proceeds to a final step 320.

Typically, as indicated by an iteration step 322, where the scanner is operated for another scan, steps 316 and 318 are iterated. As the iteration continues, the processor is able to improve the accuracy of the gains derived from equation (8) and generated in step 318.

Final step 320 is substantially the same as final step 232, the processor applying the gains derived in step 318 to all the ECG signals, while locking on to the MRI reference signal.

Fig. 6 is a flowchart 400 of steps performed in implementing system 20, according to a further alternative embodiment of the present invention. First, second, and third steps 410, 412, and 414 are generally the same as steps 210, 212 and 214, except that in step 410 there is no requirement to acquire ECG signals while the scanner is inoperative, and there is no storage of reference ECG signals.

A recording step 416 is substantially the same as step 216. Typically, the recorded ECG signals are those from patches 36 and/or stationary electrodes of probe 24. As for step 216, in step 416 the processor also acquires and records the signal generated in sensor 54, the MRI reference signal.

In a correction determining step 418 the processor, while locking on to the MRI reference signal, sums the recorded ECG signals from the patches or stationary electrodes. The summed signal provides the processor with an estimate of the noise generated by operation of the MRI sequence. The processor uses the noise estimate to generate a correction factor, i.e., a set of gains represented by equation (8), to be applied to the ECG signals, so as to reduce the MRI noise in the signals.

The flowchart then proceeds to a final step 420.

Typically, as indicated by an iteration step 422, where the scanner is operated for another scan, steps 416 and 418 are iterated. As the iteration continues, the processor is able to improve the accuracy of the gains derived from equation (8) and generated in step 418.

Final step 420 is substantially the same as final step 232, the processor applying the gains derived in step 418 to all the ECG signals, while locking on to the MRI reference signal.

In the case that two separate processors 56 and 58 are operative, and that ECG processor 58 is used to implement the steps of flowcharts 200, 300, or 400, scanner processor 56 may inform ECG processor 58 of appropriate entities associated with any given MRI sequence, such as the identity of the sequence, start and/or end times of the sequence, and start and/or end times of phases of the sequence.

Flowcharts 300 and 400 describe two different methods for operating system 20 without having a training section first. It will be appreciated that the methods described for the two flowcharts, for estimating the noise introduced into the ECG signals by the MRI scans, may be combined. Such a combination may lead to faster and/or more exact identification of the induced MRI noise, compared to applying the process of just one of the flowcharts.

It will thus be appreciated that the embodiments described above are cited by way of example, and that the present invention is not limited to what has been particularly shown and described hereinabove. Rather, the scope of the present invention includes both combinations and subcombinations of the various features described hereinabove, as well as variations and modifications thereof which would occur to persons skilled in the art upon reading the foregoing description and which are not disclosed in the prior art.

**Claims**

1.  A method, comprising:

    while imaging a patient using an initial magnetic resonance imaging (MRI) sequence, generating an initial MRI reference signal in response to the initial MRI sequence using a reference sensor placed in proximity to the patient;
    identifying, by locking on to the initial MRI reference signal, noise that will be generated in electrocardiograph (ECG) signals received from the patient;
    computing a programmable correction to be applied to the ECG signals to reduce the noise; and
    applying the programmable correction to reduce the noise in the ECG signals, received from the patient, while imaging the patient using a subsequent MRI sequence and while locking on to a subsequent MRI reference signal generated in response to the subsequent MRI sequence using the reference sensor.

2.  The method according to claim 1, and comprising placing the reference sensor so as not to pick up the ECG signals.

3.  The method according to claim 1, wherein the programmable correction comprises a set of frequency dependent gains.

4.  The method according to claim 1, wherein identifying the noise comprises analyzing the ECG signals generated from stationary electrodes coupled to the patient.

5.  The method according to claim 1, wherein identifying the noise comprises comparing the ECG signals received from the patient while not imaging the patient with an MRI sequence to the ECG signals received from the patient while imaging the patient using the initial MRI sequence.

6.  The method according to claim 1, wherein identifying the noise comprises summing the ECG signals received from the patient while imaging the patient using the initial MRI sequence.

7.  The method according to claim 6, wherein summing the ECG signals comprises summing the ECG signals generated from stationary electrodes coupled to the patient.

8.  Apparatus, comprising:

a reference sensor, placeable in proximity to a patient, and configured, while the patient is imaged using an initial magnetic resonance imaging (MRI) sequence, to generate an initial MRI reference signal in response to the initial MRI sequence; and

a processor, which is configured:

to identify, by locking on to the initial MRI reference signal, noise that will be generated in electrocardiograph (ECG) signals received from the patient,

to compute a programmable correction to be applied to the ECG signals to reduce the noise, and

to apply the programmable correction to reduce the noise in the ECG signals, received from the patient, while imaging the patient using a subsequent MRI sequence and while locking on to a subsequent MRI reference signal generated in response to the subsequent MRI sequence using the reference sensor.

9. The apparatus according to claim 8, wherein the reference sensor is placeable so as not to pick up the ECG signals.

10. The method according to claim 1 or the apparatus according to claim 8, wherein the reference sensor comprises an antenna.

11. The apparatus according to claim 8, wherein the programmable correction comprises a set of frequency dependent gains.

12. The apparatus according to claim 8, and comprising stationary electrodes coupled to the patient, and wherein identifying the noise comprises analyzing the ECG signals generated from the stationary electrodes.

13. The apparatus according to claim 8, wherein identifying the noise comprises comparing the ECG signals received from the patient while not imaging the patient with an MRI sequence to the ECG signals received from the patient while imaging the patient using the initial MRI sequence.

14. The apparatus according to claim 8, wherein identifying the noise comprises summing the ECG signals received from the patient while imaging the patient using the initial MRI sequence.

15. The apparatus according to claim 14, and comprising stationary electrodes coupled to the patient, and wherein summing the ECG signals comprises summing the ECG signals generated from the stationary electrodes.

**Patentansprüche**

1. Verfahren, umfassend:

während der Bildgebung eines Patienten das Verwenden einer anfänglichen Kernspintomographie (MRT)-Sequenz, das Erzeugen eines anfänglichen MRT-Referenzsignals als Reaktion auf die anfängliche MRT-Sequenz unter Verwendung eines in der Nähe des Patienten platzierten Referenzsensors;

das Identifizieren, durch das Aufschalten auf das anfängliche MRT-Referenzsignal, von Rauschen, das in Elektrokardiogramm (EKG)-Signalen erzeugt wird, die von dem Patienten empfangen werden;

das Berechnen einer programmierbaren Korrektur, die auf die EKG-Signale anzuwenden ist, um das Rauschen zu verringern; und

das Anwenden der programmierbaren Korrektur zur Verringerung des Rauschens in den EKG-Signalen, die von dem Patienten empfangen werden, während der Bildgebung des Patienten unter Verwendung einer nachfolgenden MRT Sequenz und während des Aufschaltens auf ein nachfolgendes MRT-Referenzsignal, das als Reaktion auf die nachfolgende MRT-Sequenz unter Verwendung des Referenzsensors erzeugt wurde.

2. Verfahren nach Anspruch 1 und umfassend das Platzieren des Referenzsensors derart, dass er die EKG-Signale nicht auffängt.

3. Verfahren nach Anspruch 1, wobei die programmierbare Korrektur einen Satz von frequenzabhängigen Verstärkungen umfasst.

4. Verfahren nach Anspruch 1, wobei das Identifizieren des Rauschens das Analysieren der EKG-Signale umfasst, die von an den Patienten gekoppelten stationären Elektroden erzeugt wurden.

**5.** Verfahren nach Anspruch 1, wobei das Identifizieren des Rauschens das Vergleichen der EKG-Signale, die von dem Patienten erhalten wurden, während keine Bildgebung des Patienten mit einer MRT-Sequenz erfolgte, mit den EKG-Signalen umfasst, die von dem Patienten während der Bildgebung des Patienten unter Verwendung der anfänglichen MRT-Sequenz erhalten wurden.

**6.** Verfahren nach Anspruch 1, wobei das Identifizieren des Rauschens das Summieren der EKG-Signale umfasst, die von dem Patienten während der Bildgebung des Patienten unter Verwendung der anfänglichen MRT-Sequenz erhalten wurden.

**7.** Verfahren nach Anspruch 6, wobei das Summieren der EKG-Signale das Summieren der EKG-Signale umfasst, die von an den Patienten gekoppelten stationären Elektroden erzeugt wurden.

**8.** Vorrichtung, umfassend:

einen Referenzsensor, der in der Nähe des Patienten platzierbar und konfiguriert ist, während der Bildgebung eines Patienten unter Verwendung einer anfänglichen Kernspintomographie (MRT)-Sequenz, ein anfängliches MRT-Referenzsignal als Reaktion auf die anfängliche MRT-Sequenz zu erzeugen; und
einen Prozessor, der konfiguriert ist:

zum Identifizieren, durch das Aufschalten auf das anfängliche MRT-Referenzsignal, von Rauschen, das in Elektrokardiogramm (EKG)-Signalen erzeugt wird, die von dem Patienten empfangen wurden,
zum Berechnen einer programmierbaren Korrektur, die auf die EKG-Signale anzuwenden ist, um das Rauschen zu verringern; und
zum Anwenden der programmierbaren Korrektur zur Verringerung des Rauschens in den EKG-Signalen, die von dem Patienten empfangen wurden, während der Bildgebung des Patienten unter Verwendung einer nachfolgenden MRT-Sequenz und während des Aufschaltens auf ein nachfolgendes MRT-Referenzsignal, das als Reaktion auf die nachfolgende MRT-Sequenz unter Verwendung des Referenzsensors erzeugt wurde.

**9.** Vorrichtung nach Anspruch 8, wobei der Referenzsensor derart platzierbar ist, dass er die EKG-Signale nicht auffängt.

**10.** Verfahren nach Anspruch 1 oder die Vorrichtung nach Anspruch 8, wobei der Referenzsensor eine Antenne umfasst.

**11.** Vorrichtung nach Anspruch 8, wobei die programmierbare Korrektur einen Satz von frequenzabhängigen Verstärkungen umfasst.

**12.** Vorrichtung nach Anspruch 8 und umfassend an den Patienten gekoppelte stationäre Elektroden, und wobei das Identifizieren des Rauschens das Analysieren der EKG-Signale umfasst, die von den stationären Elektroden erzeugt wurden.

**13.** Vorrichtung nach Anspruch 8, wobei das Identifizieren des Rauschens das Vergleichen der EKG-Signale, die von dem Patienten erhalten wurden, während keine Bildgebung des Patienten mit einer MRT-Sequenz erfolgte, mit den EKG-Signalen umfasst, die von dem Patienten während der Bildgebung des Patienten unter Verwendung der anfänglichen MRT-Sequenz erhalten wurden.

**14.** Vorrichtung nach Anspruch 8, wobei das Identifizieren des Rauschens das Summieren der EKG-Signale umfasst, die von dem Patienten während der Bildgebung des Patienten unter Verwendung der anfänglichen MRT-Sequenz erhalten wurden.

**15.** Vorrichtung nach Anspruch 14 und umfassend an den Patienten gekoppelte stationäre Elektroden, und wobei das Summieren der EKG-Signale das Summieren der EKG-Signale umfasst, die von den stationären Elektroden erzeugt wurden.

**Revendications**

**1.** Procédé consistant :

tout en imageant un patient à l'aide d'une séquence d'imagerie par résonance magnétique (IRM) initiale, à générer un signal de référence d'imagerie IRM initial à la suite de la séquence d'imagerie IRM initiale à l'aide d'un capteur de référence placé à proximité du patient ;

à identifier, en captant le signal de référence d'imagerie IRM initial, un bruit qui sera généré dans des signaux d'électrocardiographe (ECG) reçus du patient ;

à calculer une correction programmable qui doit être appliquée aux signaux ECG afin de réduire le bruit ; et

à appliquer la correction programmable afin de réduire le bruit dans les signaux ECG, reçus du patient, tout en imageant le patient à l'aide d'une séquence d'imagerie IRM ultérieure et tout en captant un signal de référence d'imagerie IRM ultérieur généré à la suite de la séquence d'imagerie IRM ultérieure à l'aide du capteur de référence.

2. Procédé selon la revendication 1, et consistant à placer le capteur de référence de sorte à ne pas détecter les signaux ECG.

3. Procédé selon la revendication 1, dans lequel la correction programmable comprend un ensemble de gains dépendant de la fréquence.

4. Procédé selon la revendication 1, dans lequel l'identification du bruit consiste à analyser les signaux ECG générés à partir d'électrodes fixes couplées au patient.

5. Procédé selon la revendication 1, dans lequel l'identification du bruit consiste à comparer les signaux ECG reçus du patient tout en n'imageant pas le patient avec une séquence d'imagerie IRM aux signaux ECG reçus du patient tout en imageant le patient à l'aide de la séquence d'imagerie IRM initiale.

6. Procédé selon la revendication 1, dans lequel l'identification du bruit consiste à sommer les signaux ECG reçus du patient tout en imageant le patient à l'aide de la séquence d'imagerie IRM initiale.

7. Procédé selon la revendication 6, dans lequel la sommation des signaux ECG consiste à sommer les signaux ECG générés à partir d'électrodes fixes couplées au patient.

8. Appareil comprenant :

un capteur de référence, qui peut être placé à proximité d'un patient et configuré, pendant que le patient est imagé à l'aide d'une séquence d'imagerie par résonance magnétique (IRM) initiale, pour générer un signal de référence d'imagerie IRM initial à la suite de la séquence d'imagerie IRM initiale ; et

un processeur, qui est configuré :

pour identifier, en captant le signal de référence d'imagerie IRM initial, un bruit qui sera généré dans des signaux d'électrocardiographe (ECG) reçus du patient ;

pour calculer une correction programmable qui doit être appliquée aux signaux ECG afin de réduire le bruit ; et

pour appliquer la correction programmable afin de réduire le bruit dans les signaux ECG, reçus du patient, tout en imageant le patient à l'aide d'une séquence d'imagerie IRM ultérieure et tout en captant un signal de référence d'imagerie IRM ultérieur généré à la suite de la séquence d'imagerie IRM ultérieure à l'aide du capteur de référence.

9. Appareil selon la revendication 8, dans lequel le capteur de référence peut être placé de sorte à ne pas détecter les signaux ECG.

10. Procédé selon la revendication 1 ou appareil selon la revendication 8, dans lequel le capteur de référence comprend une antenne.

11. Appareil selon la revendication 8, dans lequel la correction programmable comprend un ensemble de gains dépendant de la fréquence.

12. Appareil selon la revendication 8, et comprenant des électrodes fixes couplées au patient et dans lequel l'identification du bruit consiste à analyser les signaux ECG générés à partir des électrodes fixes.

**13.** Appareil selon la revendication 8, dans lequel l'identification du bruit consiste à comparer les signaux ECG reçus du patient tout en n'imageant pas le patient avec une séquence d'imagerie IRM aux signaux ECG reçus du patient tout en imageant le patient à l'aide de la séquence d'imagerie IRM initiale.

**14.** Appareil selon la revendication 8, dans lequel l'identification du bruit consiste à sommer les signaux ECG reçus du patient tout en imageant le patient à l'aide de la séquence d'imagerie IRM initiale.

**15.** Appareil selon la revendication 14, et comprenant des électrodes fixes couplées au patient et dans lequel la sommation des signaux ECG consiste à sommer les signaux ECG générés à partir des électrodes fixes.

FIG. 1

FIG. 2

FIG. 3

200

START

210 ATTACH ECG PATCHES AND INSERT PROBE INTO HEART. ACQUIRE ECG SIGNALS WHILE MRI SCANNER IS INOPERATIVE. STORE ECG SIGNALS AS REFERENCES

212 POSITION ANTENNA SO THERE IS NO PICKUP FROM ACQUIRED ECG SIGNALS

214 STORE CHARACTERISTICS OF DIFFERENT MRI SEQUENCES USED BY MRI SCANNER. SELECT MRI SEQUENCE.

216 OPERATE SCANNER WITH SELECTED SEQUENCE. RECORD ECG SIGNALS. RECORD MRI REFERENCE SIGNAL PICKED UP BY ANTENNA

224 SELECT ANOTHER MRI SEQUENCE

USE MRI REFERENCE SIGNAL TO LOCK ON TO ECG SIGNALS. WHILE LOCKED ON, USE ECG REFERENCE SIGNALS TO DETERMINE CORRECTION FACTOR TO REDUCE MRI NOISE IN ECG SIGNALS. STORE CORRECTION FACTOR

220

218

222

NO

ALL MRI SEQUENCES TESTED ?

202

YES

230 BEGIN OPERATIONAL SECTION. IDENTIFY MRI SEQUENCE USED

232 COMPUTE GAIN FOR MRI SEQUENCE FROM SEQUENCE CORRECTION FACTOR. APPLY COMPUTED GAIN TO ECG SIGNAL WHILE LOCKING ON TO MRI REFERENCE SIGNAL

204

END

FIG. 4

300

START

310 ATTACH ECG PATCHES AND INSERT PROBE INTO HEART. ACQUIRE ECG SIGNALS WHILE MRI SCANNER IS INOPERATIVE. STORE ECG SIGNALS AS REFERENCES

312 POSITION ANTENNA SO THERE IS NO PICKUP FROM ACQUIRED ECG SIGNALS

314 STORE CHARACTERISTICS OF DIFFERENT MRI SEQUENCES USED BY MRI SCANNER. SELECT MRI SEQUENCE.

316 OPERATE SCANNER WITH SELECTED SEQUENCE. RECORD ECG SIGNALS. RECORD MRI REFERENCE SIGNAL PICKED UP BY ANTENNA

322 PERFORM ANOTHER SCAN

318 USE MRI REFERENCE SIGNAL TO LOCK ONTO ECG SIGNALS. WHILE LOCKED ON USE ECG REFERENCE SIGNAL TO DETERMINE CORRECTION FACTOR TO REDUCE MRI NOISE IN ECG SIGNALS. STORE CORRECTION FACTOR

320 COMPUTE GAIN FOR MRI SEQUENCE FROM SEQUENCE CORRECTION FACTOR. APPLY COMPUTED GAIN TO ECG SIGNAL WHILE LOCKING ON TO MRI REFERENCE SIGNAL

END

FIG. 5

400

START

410 ATTACH ECG PATCHES AND INSERT PROBE INTO HEART

412 POSITION ANTENNA SO THERE IS NO PICKUP FROM ACQUIRED ECG SIGNALS

414 STORE CHARACTERISTICS OF DIFFERENT MRI SEQUENCES USED BY MRI SCANNER. SELECT MRI SEQUENCE.

416 OPERATE SCANNER WITH SELECTED SEQUENCE. RECORD ECG SIGNALS . RECORD MRI REFERENCE SIGNAL PICKED UP BY ANTENNA

422 PERFORM MORE SCANS

418 USE MRI REFERENCE SIGNAL TO LOCK ON TO ECG SIGNALS. WHILE LOCKED ON, SUM DIFFERENT LEAD ECG SIGNALS TO DETERMINE MRI NOISE. DETERMINE CORRECTION FACTOR TO REDUCE MRI NOISE IN ECG SIGNALS. STORE CORRECTION FACTOR

420 COMPUTE GAIN FOR MRI SEQUENCE FROM SEQUENCE CORRECTION FACTOR. APPLY COMPUTED GAIN TO ECG SIGNAL WHILE LOCKING ON TO MRI REFERENCE SIGNAL

END

FIG. 6

## REFERENCES CITED IN THE DESCRIPTION

**Patent documents cited in the description**

- US 6873869 B, Fischer
- US 5217010 A, Tsitlik
- US 20040225210 A, Brosovich
- US 7039455 B, Brosovich
- US 7286871 B, Cohen
- US 4991580 A, Moore
- US 6070097 A, Kreger
- EP 1872715 A, Uutela Kimmo
- WO 2012170119 A, Schweitzer
- US 4991587 A, Blakeley

- US 5391199 A
- US 5443489 A
- US 6788967 B
- US 6690963 B
- US 5558091 A
- US 6172499 B
- US 6177792 B
- US 5983126 A
- US 6456864 B
- US 5944022 A

**Non-patent literature cited in the description**

- **PRABHAKAR RAJIAH.** Cardiac MRI: Part 2, Pericardial Diseases. *American Journal of Roentgenology,* October 2011, vol. 197 (4